# EUROPÄISCHE PATENTSCHRIFT

(11) **EP 1 437 340 B1**
(45) Veröffentlichungstag und Bekanntmachung des Hinweises auf die Patenterteilung: **14.07.2010**
(21) Anmeldenummer: 03029845.9
(22) Anmeldetag: 24.12.2003
(51) Int. Cl.: C07C 209/10, C07C 209/36, C07C 211/55, C07C 211/56

(54) **Verfahren zur Herstellung von Aminodiphenylaminen**
Process for the preparation of aminodiphenylamines
Procédé de préparation d'aminodiphénylamines

(30) Priorität: 07.01.2003 DE 10300126
(43) Veröffentlichungstag der Anmeldung: 14.07.2004
(73) Patentinhaber: LANXESS Deutschland GmbH, 51369 Leverkusen (DE)
(72) Erfinder: Haider, Joachim, Dr., 50674 Köln (DE); Scholz, Ulrich, Dr., 45475 Mülheim an der Ruhr (DE); Sicheneder, Adolf, Dr., 25551 Hohenlockstedt (DE)

(56) Entgegenhaltungen:
- WO-A-02/085838
- DE-A- 2 633 811
- DE-A- 3 246 151
- US-B2- 6 316 673
- GUJADHUR R ET AL: "Formation of aryl ?nitrogen bonds using a soluble copper(I) catalyst" TETRAHEDRON LETTERS, ELSEVIER SCIENCE PUBLISHERS, AMSTERDAM, NL, Bd. 42, Nr. 29, 16. Juli 2001 (2001-07-16), Seiten 4791-4793, XP004247353 ISSN: 0040-4039

## Beschreibung

Die Erfindung betrifft ein Verfahren zur Herstellung von Nitrodiphenylaminen durch Umsetzung von gegebenenfalls substituierten Nitrohalogenbenzolen oder gegebenenfalls substituierten aktivierten Nitrobenzolen, die einen Trifluormethansulfonsäureester- oder einen Nonafluorbutansulfonsäureester- oder einen Carbamatsubstituenten enthalten, mit Anilinen, die gegebenenfalls o-, m,- oder p- substituiert sind, in Gegenwart einer Base und eines Katalysators.

4-Aminodiphenylamin (4-ADPA) ist ein wichtiges Vorprodukt zur Synthese von Alterungsschutzmitteln und Stabilisatoren in der Gummi- und Polymerindustrie (Kirk-Othmer, Encyclopedia of Chemical Technology, 4thEdition, 1992, Vol 3, Seite 424-456; Ullmann's Encylopedia of Industrial Chemistry, 5th Edition, Vol A3, 1985, Seite 91-111).

4-ADPA kann nach verschiedenen Methoden hergestellt werden. Eine Möglichkeit, 4-ADPA herzustellen, ist die zweistufige Umsetzung von Anilin bzw. Anilinderivaten mit p-Nitrochlorbenzol in Gegenwart eines Säureakzeptors oder eines Neutralisierungsmittels, gegebenenfalls in Gegenwart eines Katalysators. Die Herstellung nach dieser Methode ist beispielsweise beschrieben in DE-A 3 246 151, DE-A 3 501 698, DE-A 185 663, US-A 4 670 595, US-A 4 187 249, US-A 4 683 332 und US-A 4 187 248. Die erste Stufe wird meist mit Kupfer-Katalysatoren, die zweite mit davon unterschiedlichen Metallkomponenten, z.B. Nickel, durchgeführt (siehe hierzu z.B. US-A 5 840 982). Umsetzungen von z.B. auch halogenierten Nitrobenzolen mit Aminen in Gegenwart von Palladium-Katalysatoren sind beschrieben in US-A 5 576 460 und EP-A 846 676.

Ein Nachteil der oben angeführten Herstellungsmethode unter Kupferkatalyse ist die vergleichsweise schlechte Selektivität der Reaktion in Bezug auf Bildung des gewünschten Diarylamins. Die normalerweise dabei entstehenden 5-15 % des Triarylamins, führen sowohl zu erhöhtem destillativen Aufwand bei der Reinigung des Endproduktes, wie auch zu ökonomischen Einbußen durch erhöhten Verbrauch der Wertkomponente Halogennitrobenzol, da je Äquivalent gebildetes Triarylamin zwei Äquivalente Halogennitrobenzol verbraucht werden. Weiterhin stellt die Entsorgung des Nebenproduktes ein ökologisches Problem dar.

Es war daher wünschenswert, ein Verfahren zur Herstellung von Nitrodiphenylaminen zur Verfügung zu stellen, das von Anilinen und durch Umsetzung mit entsprechenden Nitrohalogenbenzolen ausgeht und durch anschließende Reduktion des gebildeten Nitrodiphenylamins in höherer Ausbeute und unter reduzierter Bildung des Triarylamin-Produkts zu den gewünschten Aminodiphenylaminen führt.

Venkataraman et al. [Tetrahedron Letters, 2001, 42, 4791-4793] konnten zeigen, dass die Verwendung eines präformierten Komplexes aus Kupferdibromid und Triphenylphosphin einen neuartigen Katalysator ergibt, der die Addition von Arylhalogeniden an sekundäre aromatische Amine zum Triarylamin katalysiert.

Die Addition von Arylhalogeniden an primäre Amine ergibt jedoch, wie in einem Vergleichsbeispiel gezeigt, keine signifikanten Vorteile gegenüber dem aus DE 3 246 151 A1 bekannten Cu/Cs-Katalysatorsystem.

DE 32 46 151 A betrifft ein Verfahren zur Herstellung von 4-Nitrodiphenylaminen in Gegenwart von Kaliumcarbonat und Kupferverbindungen, wobei zusätzlich Rubidium- oder Cäsiumverbindungen oder Mischungen davon eingesetzt werden. Als Kupferverbindungen werden verschiedene Kupfersalze genannt sowie organische und anorganische Koordinationsverbindungen von ein- oder zweiwertigem Kupfer, die jedoch nicht weiter präzisiert werden. Gemäß den Bespielen in DE 32 46 151 A wird als Kupferverbindung Kupfer(II)-Oxid in Kombination mit Cäsiumsalz eingesetzt.

Gujadhur et al.: Tetrahedron letters, elsevier science publishers, Amsterdam, NL, Bd. 42, Nr. 29, 16.07.2001, Seiten 4791-4793, betrifft die Synthese von funktionalisierten Biaryl- und Triarylaminen unter Verwendung eines Kupfer(I)-Komplexes, Cu(PPh₃)₃Br, als Katalysator und Cäsiumcarbonat als Base. Andere Katalysatorsysteme werden dort nicht erwähnt.

Es war daher überraschend, dass der Einsatz bestimmter Kupfer-Phosphin-Komplexe bei der Umsetzung von Nitrohalogenbenzolen mit Anilinen nur in sehr geringen Mengen das unerwünschte Triarylamin als Nebenprodukt liefert. Ebenfalls überraschend war die gesteigerte katalytische Aktivität dieser Komplexe hinsichtlich der Bildung von Nitrodiphenylaminen.

Gegenstand der vorliegenden Erfindung ist daher ein Verfahren zur Herstellung von Nitrodiphenylaminen durch Umsetzung von gegebenenfalls unsubstituierten Nitrohalogenbenzolen oder gegebenenfalls substituierten aktivierten Nitrobenzolen, die einen Trifluormethansulfonsäureester- oder einen Nonafluorbutansulfonsäureester oder Carbamatsubstituenten enthalten, mit Anilinen, die gegebenenfalls o-, m-, oder p-substituiert sind, in Gegenwart einer Base und eines Katalysators, wobei als Katalysator Kupfer-Phosphin-Komplexe eingesetzt werden, mit Phosphin-Liganden ausgewählt aus 5,5'-Dichloro-6,6'-dimethoxy-biphenyl-2,2'-diyl-bis-diphenylphosphin, Bis(2-dicyclohexylphosphino)-2'-(N,N-dimethyl-amino)-biphenyl, 2-(Dicyclohexylphosphino)biphenyl, 2-(Dicyclohexylphosphino)-2'-methylbiphenyl, 2-(Di-tert.-butylphosphino)biphenyl und 2-(Bisdiphenylphosphino)binaphthyl, und in denen das Kupfer die Wertigkeit 0, +I oder +II besitzt und die komplexierenden Kupferverbindungen ausgewählt sind aus Kupferoxiden, Kupferhalogeniden, Kupfercyaniden und Kupferacetaten, Kupferactylacetonaten in fluorierter oder nichtfluorierter Form, Kupfernitraten, Kupfertrifluormethansulfonaten, Kupferarylsulfonaten, Kupferoxinaten, Kupferphosphaten sowie Kupferpulver.

Insbesondere bevorzugt kommen als Kupfer-Phosphin-Komplexe solche infrage mit 2-(Di-tertiär-butylphosphino)biphenyl- und 2-(Dicyclohexylphosphino)biphenyl-Liganden und Kupfer(I)-bromid oder -trifluormethansulfonat.

Die genannten Kupfer-Phosphin-Komplexe können sowohl einzeln als auch im beliebigen Gemisch untereinander eingesetzt werden. Die günstigste Gemischzusammensetzung kann durch entsprechende Vorversuche bestimmt werden.

Die erfindungsgemäß einzusetzenden Kupfer-Phosphor-Komplexe werden durch Umsetzung der vorgenannten Phosphor-Verbindungen mit den vorgenannten KupferVerbindungen hergestellt.

Zur Herstellung der Kupfer-Phosphin-Komplexe werden die Einsatzprodukte der Phosphor-Verbindungen und der Kupferverbindungen in einem solchen molaren Verhältnis eingesetzt, dass sich der gewünschte Zielkomplex ergibt. Im Allgemeinen beträgt das molare Verhältnis der Phosphor-Verbindungen zu den KupferVerbindungen etwa 40:1 bis 0,5:1, bevorzugt 5:1 bis 1:1, besonders bevorzugt 4:1 bis 1:1.

Die Kupfer-Phosphin-Komplexe können separat in einem dafür geeigneten inerten organischen Lösungsmittel hergestellt werden, wie Tetrahydrofuran, Diethylether, Toluol, Xylol, Chloroform, Dichlormethan, Methanol und/oder Ethanol.

Die günstigste Menge an einzusetzendes Lösungsmittel kann durch entsprechende Vorversuche bestimmt werden.

Die Herstellung der Kupfer-Phosphin-Komplexe aus den beschriebenen Ausgangsverbindungen erfolgt durch einfaches Zusammengeben der beiden Ausgangsverbindungen in Lösung bei Raumtemperatur.

Selbstverständlich ist es auch möglich, die Kupfer-Phosphin-Komplexe in-situ während der Umsetzung der Nitrohalogenbenzolen mit Anilinen herzustellen.

Dies bedeutet, dass man die Kupfer-Phosphin-Komplexe separat der Umsetzung von Nitrohalogenbenzolen mit Anilinen zugeben kann oder - wie erwähnt - während der gesamten Umsetzung in-situ sich endlich bilden lässt.

Die Menge an einzusetzenden Kupfer-Phosphin-Komplexe beträgt üblicherweise 0,02 Mol-% bis 10 Mol-%, bevorzugt 0,1 Mol-% bis 3 Mol-%, bezogen auf die Menge an eingesetzten Nitrohalogenbenzolen.

Als Nitrohalogenbenzole werden in das erfindungsgemäße Verfahren solche eingesetzt, in denen die Nitrogruppe bevorzugt in para-Stellung zum Halogenrest steht. Als Halogenreste kommen z.B. in Frage: Fluor, Chrom oder Brom, bevorzugt Fluor oder Chrom. Statt der Nitrohalogenbenzole können auch andere aktivierte Nitrobenzole eingesetzt werde, z.B. solche die eine Trifluormethansulfonsäureester- oder eine Nonafluorbutansulfonsäureester- oder einen Carbamatsubstituenten enthalten.

Selbstverständlich können die Nitrohalogenbenzole als auch die anderen aktivierten Nitrobenzole ein- oder mehrfach substituiert sein, beispielsweise durch Alkylreste, bevorzugt solche die 1 bis 12 C-Atome, insbesondere 1 bis 4 C-Atome, enthalten. Selbstverständlich kann die Stellung der Nitrogruppe zu den Halogenresten bzw. zu den aktivierten Gruppen auch eine andere als die para-Position sein, beispielsweise eine ortho- oder meta-Position.

Als Nitrohalogenbenzole sind beispielsweise zu nennen: 4-Nitro-2-methylchlorbenzol, 4-Nitro-3-methylfluorbenzol, 4-Nitrochlorbenzol, 3-Nitrochlorbenzol oder 2-Nitrochlorbenzol, bevorzugt 4-Nitrochlorbenzol.

Als aktivierte Nitrobenzole sind z.B. zu nennen: 4-Nitrophenyl-trifluormethansulfonsäureester, 4-Nitrophenyl-nonafluorbutansulfon-säureester oder 4-Nitrophenylcarbamat, bevorzugt 4-Nitrophenyl-trifluormethylsulfonsäureester.

Als Aniline können in das erfindungsgemäße Verfahren neben Anilin selbst auch o-, m- und p-substituierte Aniline eingesetzt werden. Als Substituenten kommen beispielsweise in Frage: Verzweigte oder unverzweigte C₁-C₂₉-Alkyl- oder C₁-C₂₉-Alkenyl-Reste, Acyl-, Alkylthio-, Alkyl-amino oder Alkoxyreste mit 1 bis 29 Kohlenstoffatomen, Carbonsäureester mit 1 bis 29 Kohlenstoffatomen im Carbonsäureteil und 1 bis 29 Kohlenstoffatome im Esterteil sowie Sulfonsäurereste mit 1 bis 9 Kohlenstoffatomen im Esterteil. Insbesonders sind als Substituenten zu nennen: Verzweigte oder unverzweigte Alkyl-, Alkenyl- oder Alkylthio-Resten mit den genannten Zahlen an Kohlenstoffatomen. Zum Beispiel Octyl, Decyl, Dodecyl, Oleyl, Myristyl oder Stearyl-Reste.

Insbesondere werden als substituierte Aniline genannt: Vinylanilin, 4-tert. Butylanilin, p-Anisidin, o-Anisidin, o-Toluidin, p-Toluidin, Anthranilsäuremethylester, o-Aminobenzonitril, p-Aminobenzonitril und 4-Ethylanilin. Bevorzugt wird Anilin eingesetzt.

Gemäß dem erfindungsgemäßen Verfahren werden im Allgemeinen pro Mol Nitrohalogenbenzol oder pro Mol aktiviertes Nitrobenzol 1 bis 10 Mol, bevorzugt 1,5 bis 8 Mol, besonders bevorzugt 2 bis 6 Mol, des entsprechenden Anilins eingesetzt.

Als Basen werden im erfindungsgemäßen Verfahren Alkali- und/oder Erdalkalimetallcarbonate, -alkoholate, -phosphate, -fluoride und/oder -hydroxide eingesetzt, wobei insbesondere Kaliumcarbonat, Natriumcarbonat, Cäsiumcarbonat, Cäsiumhydrogencarbonat, Natriummethanolat, Kalium-tertiär-butylat, Kaliumamylat, Cäsiumfluorid, Kaliumphosphat und Bariumhydroxid zu nennen sind. Bevorzugt werden Kaliumcarbonat, Natriumcarbonat, Cäsiumcarbonat und/oder Cäsiumhydrogencarbonat, eingesetzt.

Besonders bevorzugt wird Kaliumcarbonat eingesetzt.

Die Basen können in unterstöchiometrischer Menge auch im Überschuss bis zur zehnfachen äquivalenten Menge bezüglich des Nitrohalogenbenzols eingesetzt werden. Besonders bevorzugt werden die Basen in der 0,3 bis 2 äquivalenten Menge, bezogen aufNitrohalogenbenzol, eingesetzt.

Für das erfindungsgemäße Verfahren ist es von Vorteil, wenn die eingesetzten Basen durch Mahlung und/oder Trocknung vorbehandelt werden.

Die Mahlung kann im erfindungsgemäßen Verfahren beispielsweise in handelsüblichen Mühlen erfolgen. Die Maßnahme des Mahlens bewirkt hierbei eine drastische Vergrößerung der spezifischen Oberfläche, die zu einer deutlichen Steigerung des Umsatzes führt. In vielen Fällen ist durch die Mahlung eine Vergrößerung der spezifischen Oberfläche um den Faktor 10 bis 20 zu beobachten.

Nach der Mahlung liegen die spezifischen Oberflächen der Basen bei ca. 0,1 bis 10 m²/g, bevorzugt 0,2 bis 1 m²/g (BET).

Aufgrund der ausgeprägten hygroskopischen Eigenschaften der im erfindungsgemäßen Verfahren eingesetzten Basen, neigen vor allem die Phosphate und Carbonate zur mehr oder minder starken Aufnahme atmosphärischer Bestandteile, wie Wasser und Kohlendioxid. Ab einer Aufnahme von ca. 30 Gewichtsprozent an atmosphärischen Bestandteilen ist ein deutlicher Einfluss auf die zu erreichenden Umsätze feststellbar. Daher ist neben der Mahlung häufig auch eine Trocknung der Basen angezeigt.

Die Trocknung der Basen erfolgt dabei je nach Natur der verwendeten Base beispielsweise derart, dass unter vermindertem Druck von ca. 0,01 bis 100 mbar für mehrere Stunden auf Temperaturen von ca. 50 bis 200°C, bevorzugt 100 bis 160°C, erhitzt wird.

Die erste Stufe des erfindungsgemäßen Verfahrens kann bei Temperaturen im Bereich von 20 bis 250°C, bevorzugt bei Temperaturen von 110 bis 210°C, durchgeführt werden. Die Reaktionstemperaturen hängen dabei insbesondere von der Art der Ausgangsprodukte, des Katalysators und der eingesetzten Basen ab.

Das erfindungsgemäße Verfahren kann sowohl in Anwesenheit als auch in Abwesenheit eines geeigneten Lösungsmittels durchgeführt werden. Als Lösungsmittel kommen beispielsweise inerte, organische Kohlenwasserstoffe, wie Xylol und Toluol, in Frage. Weiterhin können die eingesetzten aromatischen Amine selbst als Lösungsmittel fungieren.

Bei dem erfindungsgemäßen Verfahren kann - wenn gewünscht - analog zu DE-A 2 633 811 und DE-A 3 246 151 das gegebenenfalls entstehende Reaktionswasser beispielsweise durch Zuhilfenahme eines geeigneten Schleppmittels durch Destillation entfernt werden.

Die Menge der eingesetzten Lösungsmittel kann leicht durch entsprechende Vorversuche bestimmt werden.

Die Durchführung des erfindungsgemäßen Verfahrens kann nach üblichen Methoden in kontinuierlicher oder diskontinuierlicher Weise erfolgen.

Die Reduktion bzw. Hydrierung der erhaltenen Nitrodiphenylamine zu den Aminodiphenylaminen kann unter Zuhilfenahme eines Reduktionsmittels, wie Wasserstoff, gegebenenfalls in Gegenwart des bereits vorhandenen Kupfers durchgeführt werden, gegebenenfalls unter Zusatz eines geeigneten, inerten Katalysatorträgers.

Selbstverständlich ist es auch möglich, die Hydrierung in Gegenwart zusätzlicher Hydrierkatalysatoren, wie solche auf Nickel-, Palladium- oder Platin-Basis, durchzuführen, gegebenenfalls unter Einsatz eines geeigneten Katalysatorträgers.

Geeignete Materialien für die Verwendung als Katalysatorträger sind alle technisch üblichen Katalysatorträger auf der Basis von Kohlenstoff, Elementoxiden, Elementcarbiden oder Elementsalzen in verschiedenen Anwendungsformen. Beispiele für kohlenstoffhaltige Träger sind Koks, Graphit, Ruß oder Aktivkohlen. Beispiele für die Elementoxid-Katalysatorträger sind SiO₂ (natürliche oder synthetische Kieselsäure, Quarz) Al₂O₃ (α, γ-Al₂O₃), Tonerden, natürliche oder synthetische Alumosilicate (Zeolithe), Schichtsilikate wie Bentonit und Montmorillonit, TiO₂ (Rutil, Anatas), ZrO₂, MgO oder ZnO. Beispiele für Elementcarbide und -salze sind SiC, AlPO₄, BaSO₄, CaCO₃. Grundsätzlich können sowohl synthetische Materialien als auch Träger aus natürlichen Quellen, wie Bimsstein, Kaolin, Bleicherden, Bauxite, Bentonite, Kieselgur, Asbest oder Zeolithe, verwendet werden.

Bevorzugt werden Aktivkohlen und Si-, Al-, Mg-, Zr- und Ti-haltige Materialien als Trägermaterialien eingesetzt.

Besonders bevorzugt ist Aktivkohle.

Selbstverständlich kann die Hydrierung auch mit anderen Reduktionsmethoden, wie sie dem Fachmann bekannt sind und z.B. in "Reductions in Organic Chemistry, Second Edition, ACS Monograph 188" aufgeführt sind, durchgeführt werden.

Die Temperaturen bei der Hydrierung betragen ca. 0 bis 200°C, insbesondere 40 bis 150°C; die Drücke (Wasserstoffdruck) liegen bei ca. 0,1 bis 150 bar, insbesondere 0,5 bis 70 bar, ganz besonders bevorzugt 1 bis 50 bar.

Nach dem erfindungsgemäßen Verfahren werden die entsprechenden Aminodiphenylamine mit hoher Selektivität (>95%) und Ausbeuten bis zu 97 % d. Th. erhalten.

### Beispiele

### Herstellung der Kupfer-Phosphin-Komplexe

### Beispiel 1

### Herstellung von Bis(2-(di-tert-butylphosphino)biphenyl-kupfer(I)bromid)

50 ml entgastes, wasserfreies Methanol wurde auf Rückflusstemperatur erhitzt, 2,36 g (7,9 mmol) 2-(Di-tert.-butylphosphino)biphenyl wurde langsam dem Methanol zugegeben, bis die Phosphin-Verbindung vollständig gelöst war. Anschließend wurde 0,59 g (2,6 mmol) Kupfer(II)-bromid portionsweise der Lösung zugegeben. Nach Zugabe des Kupferbromids wurde die Lösung noch weitere 15 min lang auf Rückflusstemperatur erhitzt und danach die Lösung abgekühlt. Nach Abkühlen der Lösung fiel ein Feststoff aus, der abfiltriert wurde und mit wenig Ethanol und Diethylether gewaschen und anschließend getrocknet wurde. Man erhielt 0,93 g (1,1 mmol) der oben genannten Verbindung. Die Ausbeute betrug 80 % d. Th.

### Beispiel 2 (Vergleichsbeispiel)

### Herstellung von 3-[(Diphenylphosphino)oxy]phenyl diphenylphosphonitkupfer(I)chlorid

In einem Rundkolben werden 5 g (10,4 mmol) 3-[(Diphenylphosphino)oxy]phenyl diphenylphosphonit in entgastem, wasserfreiem Dichlormethan gelöst und auf 40°C ewärmt. 0,35 g Kupfer(I)chlorid (0,35 mmol) werden zugegeben. Nach 30 Minuten rühren wird das Lösungsmittel im Vakuum abgezogen. Erhalten wurde die o.g. Verbindung.

### Beispiel 3 (Vergleichsbeispiel)

### Herstellung von dimeren 1,1'-Biphenyl-2-yl-dicyclohexylphosphonit-Kupfer(I)chlorid

In einem Rundkolben werden 5g (13,6mmol) 1,1'-Biphenyl-2-yl-dicyclohexylphosphonit in wasserfreiem, entgastem Chloroform gelöst. Im Argongegenstrom werden 0,45g (4,5mmol) Kupfer(I)chlorid zugegeben und 6h bei Raumtemperatur gerührt. Das Lösungsmittel wird im Vakuum entfernt und der Rückstand in wasserfreiem Ether aufgenommen. Man kühlt auf -78°C ab, dabei fällt das Produkt aus.

### Beispiel 4 (Vergleichsbeispiel)

### Herstellung von dimeren 4-Isopropoxydinaphtho[2,1-d:1',2'-f][1,3,2]dioxaphosphepin - Kupfer(I) chlorid

In einem Rundkolben werden 5 g (13,4 mmol) 4-Isopropoxydinaphtho[2,1-d:1',2' f][1,3,2]dioxa-phosphepin (rac.-Binaphthyl-isopropyl-phosphit) in Dichlormethan gelöst und im Argongegenstrom 0,44 g (0,45 mmol) Kupfer(I)chlorid zugegeben. Nach 30 Minuten rühren wird das Lösungsmittel im Vakuum abgezogen.

### Beispiel 5

### 4-NDPA-Kondensation mit in situ erzeugtem Cu-Phosphin-Katalysator mit Cu(I)-Bromid als Vorstufe

In einem 1000 ml Vierhalskolben mit mechanischem Rührer, aufgesetzter Vigreux-Kolonne und Wasserabscheider (mit 35ml Xylol gefüllt) wurden 288,9 g (3090 mmol) Anilin, 8,16 g (27 mmol) 2-(Di-t-butylphosphino)biphenyl, 3,87 g (27 mmol) Cu(I)bromid, 83,6 g (605 mmol) gemahlenes Kaliumcarbonat und 157,6 g (1000 mmol) p-NCB unter Rühren und N₂-Atmosphäre in das Reaktionsgefäß gegeben und auf Rückflusstemperatur erhitzt. Die anfangs geringe Wasserentwicklung steigerte sich im Verlauf der Reaktion und blieb dann auf niedrigem Niveau konstant. (insg. ca. 9 ml). Die Temperatur des Gemisches stieg von anfangs 189°C auf 200°C. Alle 30 Min. wurden Proben gezogen (unfiltriert) und der Umsatz und das 4-NDPA/Triarylamin-Verhältnis mit HPLC bestimmt. Nach 4,5 h wurde der Versuch beendet. Dabei ergab sich ein Rest-pNCB-Wert von 0,66 Gew.-% (entspricht 99 % Umsatz an pNCB), ein 4-NDPA-Gehalt von 41,2 Gew.-%, der 4,4'-Dinitrotriphenylamin-Gehalt betrug 0,78 % und das 4-NDPA-/Triarylamin-Verhältnis von 52:1 (entspricht 97 % d.Th an 4-NDPA und eine 98 %ige Selektivität, bezogen auf p-NCB).

### Beispiel 6

### 4-NDPA-Kondensation mit präformiertem Cu₂Br₂(2-(Di-t-butylphosphino)-biphenyl)₂-Katalysator (reduzierte Menge)

In einem 1000 ml Vierhalskolben mit mechanischem Rührer, aufgesetzter Vigreux-Kolonne und Wasserabscheider (mit 35 ml Xylol gefüllt) wurden 3,22 g (3,64 mmol) Cu₂Br₂(2-(Di- t-butylphosphino)biphenyl)₂ unter 50l/h N₂-Strom bei Raumtemperatur für 10 min in 372 g (3,99 mol) Anilin gerührt. Anschließend 157,6 g (1,00 mol) para-Nitrochlorbenzol (Abkürzung p-NCB) und 96,6 g (700 mmol) gemahlenes Kaliumcarbonat zugegeben und unter Rückfluss aufgeheizt. Bei Beginn des Rückflusses setzt eine konstante Wasserabscheidung ein. Die Temperatur des Gemisches stieg von anfangs 189°C auf 196°C. Nach 5h wurde die Zusammensetzung des Reaktionsgemisches durch Probennahme (unfiltriert) mit HPLC bestimmt und das 4-NDPA/Triarylamin-Verhältnis mit HPLC bestimmt und der Reaktionsansatz abgekühlt. Dabei ergab sich ein Rest-pNCB-Wert von 6,2 Gew.-% (entspricht 84 % Umsatz), ein 4-NDPA-/Triarylamin-Verhältnis von 59 (entspricht 82,4 % 4-NDPA-Ausbeute und 98 % Selektivität bezogen auf p-NCB).

### Beispiel 7

### 4-NDPA-Kondensation mit in situ erzeugtem Cu-Phosphin-Katalysator (reduzierte Menge) mit Cu(I)-Bromid als Vorstufe

In einem 1000 ml Vierhalskolben mit mechanischem Rührer, aufgesetzter Vigreux-Kolonne und Wasserabscheider (mit 35 ml Xylol gefüllt) wurden 288,9 g (3090 mmol) Anilin, 2,42 g (8,1 mmol) 2-(Di-t-butylphosphino)biphenyl, 1,16 g (8,1 mmol) Cu(I)bromid, 83,6 g (605 mmol) gemahlenes Kaliumcarbonat und 157,6 g (1000 mmol) p-NCB unter Rühren und N₂-Atmosphäre in das Reaktionsgefäß gegeben und auf Rückflusstemperatur erhitzt. Die anfangs geringe Wasserentwicklung steigerte sich im Verlauf der Reaktion und blieb dann auf niedrigem Niveau konstant. (insg. ca. 7 ml). Die Temperatur des Gemisches stieg von anfangs 189°C auf 198°C. Alle 30 Min. wurden Proben gezogen (unfiltriert) und der Umsatz und das 4-NDPA/Triarylamin-Verhältnis mit HPLC bestimmt. Nach 7h wurde der Versuch beendet. Dabei ergab sich ein Rest-pNCB-Wert von 4,2 Gew.-% (entspricht 89 % Umsatz), ein 4-NDPA-Gehalt von 37,2 Gew.-%, 4,4'-Dinitrotriphenylamin-Gehalt von 1,9 % und 4-NDPA-/Triarylamin-Verhältnis von 33 (entspricht 86 % 4-NDPA-Ausbeute und 97 % Selektivität bezogen auf p-NCB).

### Beispiel 8

### 4-NDPA-Kondensation mit in situ Präparation von Cu-Phosphin-Katalysator mit Cu(I)-Triflat-Benzol-Komplex als Vorstufe

In einem 1000 ml Vierhalskolben mit mechanischem Rührer, aufgesetzter Vigreux-Kolonne und Wasserabscheider (mit 35 ml Xylol gefüllt) wurden 288,9 g (3090 mmol) Anilin, 2,14 g (27 mmol) Kupfer-(II)-oxid, 83,6 g (605 mmol) gemahlenes Kaliumcarbonat und 157,6 g (1000 mmol) p-NCB vorgelegt und dann bei einem Stickstoffstrom von ca. 150 L/h bis zum Rückfluss erhitzt. Es schied sich nach kurzer Zeit ca. 0,4 ml Wasser ab. Nach ca. 0,5 h wurde auf etwa 120°C abgekühlt, 4,53 g (8,1 mmol) Cu(I)trifluormethansulfonat Benzol-Komplex und 2,42 g (8,1 mmol) 2-(Di-*tert*.-butylphosphino)biphenyl-Ligand zugesetzt. Anschließend wurde unter leichtem N₂-Strom wieder auf Rückflusstemperatur erhitzt. Daraufhin kam es zunächst zu einer untypisch geringen Wasserentwicklung, die dann aber immer schwächer wurde und spätestens nach 2 h völlig zum Erliegen kam (ca. 0,5 ml). Die Temperatur des Reaktionsgemisches stieg von anfangs 188°C auf 196°C an. Nach 6 h wurde eine Probe gezogen und die Zusammensetzung des Reaktionsgemisches mit HPLC bestimmt. Anschließend wurde der Versuch beendet. Dabei ergab sich ein Rest p-NCB-Wert von 4,0 Gew.-% (entspricht 89 % Umsatz), ein 4-NDPA-Gehalt von 39,6 Gew.-%, 4,4'-Dinitrotriphenylamin-Gehalt von 1,2 % und ein 4-NDPA-/Triarylamin-Verhältnis von 33 (entspricht 86,4 % 4-NDPA-Ausbeute und 86 % Selektivität bezogen auf p-NCB).

### Beispiel 9

### 4-NDPA-Kondensation Cu-Phosphin-/Phosphit- und Phosphonit-Katalysator im 10 mmol Maßstab

Für das Standardsystem werden in den 10ml - Reaktionsgefäßen 1,58 g p-NCB (10 mmol), 0,95 g Pottasche (6,9 mmol), 21,5 mg Cu(II)O (0,25 mmol), 15,5 mg CsHCO3 (0,08 mmol) sowie Anilin 3,75 g (40 mmol) eingewogen. Beim Katalysatorscreening bleiben p-NCB, Pottasche und Anilin unverändert, es werden aber 0,25 mmol der verschiedenen Testkatalysatoren eingesetzt. Auf die Reaktionsgefäße werden mit Molsieb gefüllte Kühlrohre gesetzt. Die Reaktionen werden in einem Rühr-Heiz-Block bei 200°C Blocktemperatur und 530U/min durchgeführt. Nach 4 Stunden wurden die Reaktionen beendet. Nach Abkühlung um etwa 40°C wurde jeweils eine Probe gezogen und mittels HPLC analysiert.

| **Testkatalysator** | **Umsatz (p-NCB) [%]** | **Ausbeute** **(4-NDPA)** **[%]** | **Selektivität** **4-NDPA/** **Triarylamin** |
|---|---|---|---|
| Cu(II)O/CsHCO₃ (Referenz) | 70 | 67 | 22:1 |
| 1,1'-Biphenyl-2-yl ditert.-butylphosphin/ Cu-(II)-acetylacetonat | 87 | 85 | 57:1 |
| 1,1'-Biphenyl-2-yl ditert.-butylphosphin/ Cu-(II)-acetat | 86 | 85 | 63:1 |
| 1,1'-Biphenyl-2-yldicyclohexyl-phosphonit- Kupfer(I)chlorid-Dimer. (Vergleichsbeispiel) | 75 | 73 | 39:1 |
| Cu(II)O/ 2 PPh₃ (Vergleichsbsp.*) | 50 | 48 | 25:1 |

| | | | |
|---|---|---|---|
| *)analog Venkataraman et al. [Tetrahedron Letters, 2001, 42, 4791-4793] | | | |

### Vergleichsbeispiel 1 (analog DE 3 246 151 A1)

### 4-NDPA-Kondensation mit Cu(II)O-/Cs-Katalysator

In einem 1000 ml Vierhalskolben mit mechanischem Rührer, aufgesetzter Vigreux-Kolonne und Wasserabscheider (gefüllt mit 35 ml Xylol) wurden 288,9 g (3090 mmol) Anilin, 2,44 g (12,6 mmol) Cäsiumhydrogencarbonat, 2,14 g (27 mmol) Kupfer-(II)-oxid, 83,6g (605 mmol) gemahlenes Kaliumcarbonat und 157,6 g (1000 mmol) p-NCB zugegeben und die Reaktionsmischung unter Rückfluss erhitzt. Dabei wurde nur ein leichter N₂-Strom zur Aufrechterhaltung der Inertgasatmosphäre eingestellt. Ab ca. 189°C setzte die Wasserabscheidung ein, die im Fortlauf der Reaktion andauerte. Die Reaktionstemperatur stieg bis zum Ende der Reaktion nach 5,5 h auf ca. 203°C an . Die Zusammensetzung des Reaktionsgemisches wurde durch HPLC bestimmt. Dabei ergab sich ein Rest p-NCB-Wert von 0,1 Gew.-% (p-NCB-Umsatz > 99%), ein 4-NDPA-Gehalt von 44,8 Gew.-% und 4,4'-Dinitrotriphenylamin-Gehalt von 2,1 % und ein 4-NDPA-/Triarylamin-Verhältnis von 21 (entspricht 87,5 % d. Th. Ausbeute an 4-NDPA und 88 % Selektivität bezogen auf p-NCB).

### Vergleichsbeispiel 2 (analog DE 3 246 151 A1)

### 4-NDPA-Kondensation mit reduzierter Menge an Cu(II)O-/Cs-Katalysator

In einem 1000 ml Vierhalskolben mit mechanischem Rührer, aufgesetzter Vigreux-Kolonne und Wasserabscheider (gefüllt mit 35 ml Xylol) wurden 288,9 g (3090 mmol) Anilin, 733 mg (3,78 mmol) Cäsiumhydrogencarbonat 642 mg (8,1 mmol) Kupfer-(II)-oxid, 83,6g (605 mmol) gemahlenes Kaliumcarbonat und 157,6 g (1000 mmol) 4-Nitrochlorbenzol zugegeben und die Reaktionsmischung unter Rückfluss erhitzt. Dabei wurde nur ein leichter N₂-Strom zur Aufrechterhaltung der Inertgasatmosphäre eingestellt. Ab ca. 189°C setzte die Wasserabscheidung ein, die im Fortlauf der Reaktion andauerte. Die Reaktionstemperatur stieg bis zum Ende der Reaktion nach 5 h auf ca. 201°C an. Die Zusammensetzung des Reaktionsgemisches wurde durch HPLC bestimmt. Dabei ergab sich ein Rest p-NCB-Wert von 12,5 Gew.-% (entspricht 63 % Umsatz an p-NCB), ein 4-NDPA-Gehalt von 22,6 Gew.-% und 4,4'-Dinitrotriphenylamin-Gehalt von 1,1 Gew.-% und ein 4-NDPA-/Triarylamin-Verhältnis von 21 (entspricht 60 % d. Th. Ausbeute an 4-NDPA und 95 % Selektivität bezogen auf p-NCB).

### Beispiel 10 (Herstellung von 4-ADPA)

Zur Reaktionsmischung aus Beispiel 8 wurden nach Abkühlen auf 105°C 250 ml Wasser zugegeben, 15 min bei 80°C gerührt und in einen Scheidetrichter überführt. Nach erfolgter Phasentrennung wurden die abgetrennte organische Phase (500 ml) mit 5ml KOH, 25 ml Salzwasser (aus wässriger Phase der Kondensation) und 4,5 g Raney-Nickel versetzt und in einen Hydrierautoklav überführt und bei einem Druck von 10 bar Wasserstoff in 400 min hydriert, wobei die Temperatur von 140°C erreicht wurde. Nach gaschromatographischer Untersuchung erhält man 99% 4-Aminodiphenylamin (bezogen auf das eingesetzte 4-Nitrodiphenylamin).

## Patentansprüche

1. Verfahren zur Herstellung von Nitrodiphenylaminen, durch Umsetzung von gegebenenfalls substituierten Nitrohalogenbenzolen oder gegebenenfalls substituierten aktivierten Nitrobenzolen, die einen Trifluormethansulfonsäureester- oder einen Nonafluorbutansulfonsäureester- oder einen Carbamatsubstituenten enthalten, mit Anilinen, die gegebenenfalls o-, m,- oder p-substituiert sind, in Gegenwart einer Base und eines Katalysators, **dadurch gekennzeichnet, dass** als Katalysator Kupfer-Phosphin-Komplexe eingesetzt werden, mit Phosphin-Liganden, ausgewählt aus 5,5'-Dichloro-6,6'-dimethoxybiphenyl-2,2'-diyl-bis-diphenylphosphin, Bis(2-dicyclohexylphosphino)-2'-(N,N-dimethyl-amino)-biphenyl, 2-(Dicyclohexylphosphino)biphenyl, 2-(Dicyclohexylphosphino)-2'-methylbiphenyl, 2-(Di-tert.-butylphosphino)-biphenyl und 2-(Bisdiphenylphosphino)binaphthyl, und in denen das Kupfer die Wertigkeit 0, +I oder +II besitzt und die komplexierenden Kupferverbindungen ausgewählt sind aus Kupferoxiden, Kupferhalogeniden, Kupfercyaniden und Kupferacetaten, Kupferactylacetonaten in fluorierter oder nichtfluorierter Form, Kupfernitraten, Kupfertrifluormethansulfonaten, Kupferarylsulfonaten, Kupferoxinaten, Kupferphosphaten sowie Kupferpulver.

2. Verfahren nach Anspruch 1, **dadurch gekennzeichnet, dass** als Kupfer-Phosphin-Komplexe solche 2-(Di-tertiär-butylphosphino)biphenyl- und 2-(Dicyclohexylphosphino)biphenyl-Liganden und Kupfer(I)-bromid oder -trifluormethansulfonat eingesetzt werden.

3. Verfahren zur Herstellung von Aminodiphenylaminen durch Herstellung von Nitrodiphenylaminen in einem Verfahren gemäß Anspruch 1 oder 2 und anschließende Hydrierung der intermediär entstehenden Nitrodiphenylamine in üblicher Weise.

## Claims

1. Process for preparing nitrodiphenylamines by reacting optionally substituted nitrohalobenzenes or optionally substituted activated nitrobenzenes containing a trifluoromethanesulphonic ester substituent or a nonafluorobutanesulphonic ester substituent or a carbamate substituent with anilines which are optionally o-, m- or p-substituted in the presence of a base and a catalyst, **characterized in that** copper-phosphine complexes which have phosphine ligands selected from among 5,5-dichloro-6,6'-dimethoxybiphenyl-2,2'-diylbisdiphenylphosphine, bis(2-dicyclohexylphosphino)-2'-(N,N-dimethylamino)-biphenyl, 2-(dicyclohexylphosphino)biphenyl, 2-(dicyclohexylphosphino)-2'-methylbiphenyl, 2-(di-tert-butylphosphino)biphenyl and 2-(bisdiphenylphosphino)binaphthyl and in which the copper has the valency 0, +I or +II and the complexing copper compounds are selected from among copper oxides, copper halides, copper cyanides and copper acetates, copper acetylacetonates in fluorinated or unfluorinated form, copper nitrates, copper trifluoromethanesulphonates, copper arylsulphonates, copper oxinates, copper phosphates and copper powder are used as catalyst.

2. Process according to Claim 1, **characterized in that** complexes of 2-(di-tert-butylphosphino)biphenyl and 2-(dicyclohexylphosphino)biphenyl ligands and copper(I) bromide or trifluoromethanesulphonate are used as copper-phosphine complexes.

3. Process for preparing aminodiphenylamines by preparing nitrodiphenylamines in a process according to Claim 1 or 2 and subsequently hydrogenating the nitrodiphenylamines formed as intermediates in a conventional way.

## Revendications

1. Procédé pour la préparation de nitrodiphénylamines, par transformation de nitrohalogénobenzènes le cas échéant substitués ou de nitrobenzènes activés, le cas échéant substitués, qui contiennent un substituant ester de l'acide trifluorométhanesulfonique ou ester de l'acide nonafluorobutanesulfonique ou carbamate, avec des anilines, qui sont le cas échéant substituées en position ortho, méta ou para, en présence d'une base et d'un catalyseur, **caractérisé en ce qu'**on utilise comme catalyseur des complexes de cuivre-phosphine, avec des ligands de type phosphine, choisis parmi la 5,5'-dichloro-6,6'-diméthoxybiphényl-2,2'-diylbisdiphénylphosphine, le bis(2-dicyclohexylphosphino)-2'-(N,N-diméthylamino)-biphényle, le 2-(dicyclohexylphosphino)-biphényle, le 2-(dicyclohexylphosphino)-2'-méthylbiphényle, le 2-(di-tert-butylphosphino)-biphényle et le 2-(bisdiphénylphosphino)binaphtyle, et dans lesquels le cuivre présente la valence 0, +I ou +II et les composés de cuivre complexants sont choisis parmi les oxydes de cuivre, les halogénures de cuivre, les cyanures de cuivre et les acétates de cuivre, les acétylacétonates de cuivre sous forme fluorée et non fluorée, les nitrates de cuivre, les trifluorométhanesulfonates de cuivre, les arylsulfonates de cuivre, les oxynates de cuivre, les phosphates de cuivre ainsi que la poudre de cuivre.

2. Procédé selon la revendication 1, **caractérisé en ce qu'**on utilise comme complexes de cuivre-phosphine des ligands 2-(di-tert-butylphosphino)biphényle et 2-(dicyclohexylphosphino)biphényle et du bromure de cuivre (I) ou du trifluorométhanesulfonate de cuivre (1).

3. Procédé pour la préparation d'aminodiphénylamines par préparation de nitrodiphénylamines dans un procédé selon la revendication 1 ou 2 et hydrogénation consécutive des nitrodiphénylamines formées de manière intermédiaire de manière usuelle.
